Europäisches Patentamt

European Patent Office

Office européen des brevets

(11) Veröffentlichungsnummer: **0 072 010**
**B1**

# (12) EUROPÄISCHE PATENTSCHRIFT

(45) Veröffentlichungstag der Patentschrift: **30.09.87**

(21) Anmeldenummer: **82107157.8**

(22) Anmeldetag: **07.08.82**

(51) Int. Cl.⁴: **C 12 P 7/56**, C 12 N 1/20 // C12R1/225

(54) **Verfahren zur Herstellung von D-Milchsäure unter Verwendung von Lactobacillus bulgaricus DSM 2129.**

(30) Priorität: **11.08.81 DE 3131717**

(43) Veröffentlichungstag der Anmeldung:
**16.02.83 Patentblatt 83/07**

(45) Bekanntmachung des Hinweises auf die Patenterteilung:
**30.09.87 Patentblatt 87/40**

(84) Benannte Vertragsstaaten:
**AT BE CH DE FR GB IT LI LU NL SE**

(56) Entgegenhaltungen:
GB-A-1 157 213
THE AUSTRALIAN JOURNAL OF DAIRY TECHNOLOGY, Band 32, Nr. 1, März 1977, Seiten 19-22 G.C. COX et al.: "Lactic acid production by Lactobacillus Bulgaricus in supplemented whey ultrafiltrate"
CHEMICAL ABSTRACTS, Band 54, 25. November 1960, Spalte 25383c-d, Columbus, Ohio, USA N. OETIKER: "Manufacture of lactic acid and its use as a precipitant for casein"

(73) Patentinhaber: HOECHST AKTIENGESELLSCHAFT
Postfach 80 03 20
D-6230 Frankfurt am Main 80 (DE)

(72) Erfinder: Voelskow, Hartmut, Dr.
Eschenstrasse 24
D-6234 Hattersheim am Main (DE)
Erfinder: Sukatsch, Dieter, Dr.
Wartburgstrasse 10
D-6230 Frankfurt am Main 80 (DE)

(56) References cited:
CHEMICAL ABSTRACTS, Band 59, Nr. 6, 16. September 1963, Spalte 6901a, Columbus, Ohio, USA G. GENIN: "The use of lactic acid in the preparation of casein"

CHEMICAL ABSTRACTS, Band 87, Nr. 17, 24. Oktober 1977, Seite 515, Nr. 132339y, Columbus, Ohio, USA

Courier Press, Leamington Spa, England.

**Beschreibung**

Aus der britischen Patentschrift 1 157 213 ist ein Verfahren zur fermentativen Herstellung von D-Milchsäure bekannt. Der für dieses Verfahren eingesetzte Lactobacillus leichmannii ATCC 4797 ist in der Lage, innerhalb von 60 Stunden 113 g D-Milchsäure/l Nährmedium herzustellen, das neben anderen üblichen Zusatzstoffen entweder Glucose oder Rübenzuckermelasse enthält.

Im Hinblick auf den zunehmenden Bedarf an D-Milchsäure also Ausgangssubstanz für chemische Synthesen optisch aktiver Verbindungen stellte sich nun die Aufgabe, ein leistungsfähigeres Verfahren zu ihrer Gewinnung zu entwickeln. Wünschenswert war dabei vor allem eine Abkürzung der Fermentationszeit und das Auffinden eines Mikroorganismus, der nicht nur Glucose und Rübenzuckermelasse, sondern auch Milchzucker verwertet, der in der Molke in großen Mengen zur Verfügung steht. Dieses Ziel konnte mit Hilfe von Lactobacillus bulgaricus erreicht werden.

Das Milchsäurebakterium Lactobacillus bulgaricus ist dafür bekannt, daß es aufgrund seiner hohen Stoffwechselaktivität zu einer besonders schnellen Gärung befähig ist. Es wird deshalb zur Joghurtherstellung eingesetzt und ist in der Lage, die Milchgärung innerhalb von 4 Stunden zu beenden. Allerdings können die in der Natur und in vergorener Milch vorkommenden Stämme von Lactobacillus bulgaricus nicht mehr als 25 bis 30 g Milchsäure/l Medium produzieren. Mehr als die Hälfte des vorhandenen Milchzuckers bleibt unvergoren zurück.

Überraschenderweise wurde nun in einer angesäuerten Milchprobe ein Stamm von Lactobacillus bulgaricus gefunden, dessen Leistungsfähigkeit durch planmäßige Selektionen so weit verbessert werden konnte, daß er bis zu 115 g Milchsäure/l Medium bildet. Eine genaue Identifizierung dieses Stammes zeigte, daß er aus langen grampositiven Stäbchen besteht, die Katalase-negativ, unbeweglich und mikroaerophil sind und keine Sporen tragen. Im übrigen wird er durch folgende Daten gekennzeichnet:

Stoffwechsel: Homofermentative Milchsäuregärung

| | |
|---|---|
| Gas aus Glucose: | — |
| Gas aus Gluconat: | — |
| Wachstum bei 15°C | — |
| Wachstum bei 45°C: | + |

| Säurebildung aus | Ribose: | — |
|---|---|---|
| | Arabinose: | — |
| | Xylose: | — |
| | Mannit: | — |
| | Sorbit: | — |
| | Glucose: | + |
| | Galactose: | — |
| | Lactose: | + |
| | Maltose: | — |
| | Saccharose: | — |
| | Trehalose: | — |
| | Cellobiose: | — |
| | Melibiose: | — |
| | Raffinose: | — |
| | Salicin: | — |
| | Amygdalin: | — |

Argininspaltung: —
Konfiguration der Milchsäure: D(−)
Diaminopimelinsäure in der Zellwand: keine

Dieser Stamm wurde bei der deutschen Sammlung für Mikroorganismen unter der Nummer DSM 2129 hinterlegt.

Der neue Stamm von Lactobacillus bulgaricus ist in der Lage, innerhalb von 48 Stunden bis zu 115 g D-Milchsäure/l Medium zu bilden. Er ist außerdem nicht auf der Vergärung von Glucose spezialisiert, sondern kann Milchzucker fast ebenso schnell wie Glucose zu Milchsäure vergären. Damit kann aber auch die in großen Mengen als Abfall der Milchindustrie zur Verfügung stehenden Molke als Rohstoffquelle zur Erzeugung von D-Milchsäure eingesetzt werden.

Gegenstand der Erfindung ist deshalb auch ein Verfahren zur Herstellung von D-Milchsäure durch Fermentation eines Glucose und/oder Milchzucker und andere übliche Zusatzstoffe enthaltenden Nährmediums, bei de man als milchsäure erzeugenden Mikroorganismus Lactobacillus bulgaricus DSM 2129 einsetzt. Der Milchzucker wird dabei in der Regel in Form frischer Molke oder eine Suspension von Molkepulver eingesetzt, der gegebenenfalls auch noch Glucose zugesetzt werden kann.

Die zur Gewinnung von D-Milchsäure verwendeten Nährmedien sind in an sich bekannter Weise zusammengesetzt. Sie müssen neben Glucose und/oder Milchzucker natürlich auch noch eine Stickstoffquelle enthalten, wofür zum Beispiel Fleischextrakt, Maisquellwasser (Cornsteep) oder auch Sojamehl in Betracht kommen. Außerdem werden auch Mineralsalze, Vitamine und oberflächenaktive Mittel zugesetzt. Als oberflächenaktive Mittel kommen insbesondere handelsübliche nichtionische Tenside, vor allem flüssige Produkte, in Betracht. Diese Tenside enthalten als Wirkstoff im allgemeinen Polyoxalkylate, z.B. Umsetzungsprodukte von Alkoholen und Säuren mit Ethylenoxid und/oder Propylenoxid. Als Alkohole kommen ein- und mehrwertige in Betracht wie Fettalkohole, Harzalkohole, Glycerin, Erythrit, Pentaerythrit oder Zuckeralkohole wie Sorbit und Mannit, als Säuren vor allem Fett- und Harzsäuren. Günstig sind auch Teilester aus solchen mehrwertigen Alkoholen und den genannten Säuren wie Oxethylate eines Anhydrosorbit-monooleats.

Da Lactobacillus bulgaricus DSM 2129 säureempfindlich ist, muß die entstehende Milchsäure mit Alkali- oder Erdalkalihydroxiden oder Carbonaten, insbesondere durch Calciumcarbonat, gebunden werden. Der pH-Wert wird hierdurch im Bereich von 4, 5 bis 7 gehalten, bevorzugt 6, 5 bis 6, 8.

Jedes Nährmedium sollte vor der Beimpfung mit Lactobacillus bulgaricus DSM 2129 sterilisiert werden, um etwaige Verunreinigungen durch Fremdorganismen zu beseitigen. Hierzu reicht es aus, das Nährmedium 15 Minuten auf 121°C zu erhitzen.

Wie für alle Milchsäuregärungen sind auch für das erfindungsgemäße Verfahren anaerobe Bedingungen einzuhalten. Hierzu reicht das durch die Neutralisation der Milchsäure mit Calciumcarbonat entstehende Kohlendioxid oder eine Überlagerung des Fermentationsmediums mit Stickstoff bereits aus.

Die Milchsäuregärung kann in einem Temperaturbereich zwischen 30 und 50°C durchgeführt werden. Ein besonders günstiger Temperaturbereich liegt bei 40 bis 45°C.

Aus dem bei dem erfindungsgemäßen Verfahren anfallenden Salz der D-Milchsäure kann die freie D-Milchsäure durch Ionenaustaucher oder im Falle des Calciumlactates durch Ansäuern mit Schwefelsäure gewonnen werden.

Die Erfindung wird durch die nachfolgenden Beispiele näher erläutert. Prozentangaben beziehen sich auf das Gewicht.

Beispiel 1
Entwicklung des Stammes Lactobacillus bulgaricus DSM 2129
Der in einer Probe vergorener Milche gefundene Stamm Lactobacillus bulgaricus wurde isoliert und auf folgendem Medium 1 kultiviert (alle Angaben in g/l):

| | |
|---|---|
| Caseinpepton (tryptisch verdaut) | 10 |
| Fleischextrakt (Merck) | 10 |
| Hefeextrakt | 5 |
| Glukose | 20 |
| $K_2HPO_4$ | 2 |
| Natriumazetat | 5 |
| $MgSO_4 \cdot 7H_2O$ | 0,2 |
| $MnSO_4 \cdot H_2O$ | 0,05 |
| nichtionisches Tensid | 1 ml/l |

Danach wurden Proben des Stammes verdünnt und auf Agarplatten geimpft, die aus dem gleichen Medium mit einem Zusatz von 1,8% Agar hergestellt waren. Die beimpften Agarplatten wurden anschließend unter anaeroben Bedingungen im Brutschrank bei 45°C einen Tag aufbewahrt.

Insgesamt wurden dann 30 Einzelkolonien entnommen und jeweils in ein Kulturröhrchen gegeben, das folgendes Medium 4 enthielt (Angaben in g/l):

| | |
|---|---|
| Glucose | 50 |
| $CaCO_3$ | 70 |
| Hefeextrakt | 7 |
| Cornsteep (trocken) | 15 |
| Natriumazetat | 5 |
| nichtionisches Tensid | 1 ml/l |

Die Kulturzeit betrug 24 Stunden bei 45°C. Nach 24 Stunden war der Unterschied zwischen den verschieden gut gärenden Kulturen an den jeweils gebildeten Mengen der D-Milchsäure deutlich zu erkennen. Das Röhrchen mit dem höchsten Gehalt an D-Milchsäure wurde für einen neuen Plattenausstrich verwendet. Dieser Vorgang wurde während 8 Wochen regelmäßig wiederholt und dabei der Zuckergehalt des Mediums zunächst auf 8% und nach weiteren 5 Wochen auf 10% gesteigert. Anschließend wurde der Stamm weitere 12 Wochen lang selektioniert, bis er schließlich befähigt war, 100 g Glukose/l Medium in 24 Stunden zu 65% zu metabolisieren, wobei 55 g D-Milchsäure/l gebildet wurden. Ein vollständiger Glukoseabbau wurde nach 36 bis 40 Stunden beobachtet.

Biespiel 2
Der gemäß Beispiel 1 gewonnene Stamm von Lactobacillus bulgaricus wurde in Kulturröhrchen

3

geimpft, die jeweils 15 ml des Mediums 1 enthielten. Die Röhrchen standen zur Kultur bei 45°C für mindestens 8 Stunden und höchstens 20 Stunden senkrecht und wurden dann als Inokulum für Kulturen in Erlenmeyerkolben verwendet, die jeweils 250 ml des Mediums 1 enthielten. Diese wurden ebenfalls bei 45°C 8 bis 20 Stunden aufbewahrt.

Der Inhalt von 6 der genannten Erlenmeyerkolben diente dann als Inokulum für einen Fermenter mit 30 l Inhalt, in den vorher folgendes Medium 2 gegeben war (Angaben in g/l):

| | |
|---|---|
| Glukose | 30 |
| $CaCO_3$ | 15 |
| Hefeextrakt | 7 |
| Caseinpepton | 10 |
| Cornsteep (trocken) | 20 |
| Natriumazetat | 5 |
| nichtionisches | 1 ml/l |

Der Fermenter wird mit 100 Umdrehungen/min gerührt und nach 8 bis 12 Stunden bei 45°C in einen Fermenter mit 270 l Inhalt gegeben, der entweder das in Beispiel 1 genannte Medium 4 oder das folgende Medium 3 enthält (Angaben in g/l):

| | |
|---|---|
| Glukose | 30 |
| $CaCO_3$ | 70 |
| Hefeextrakt | 7 |
| Sojamehl | 15 |
| Natriumacetat | 5 |
| nichtionisches Tensid | 1 ml/l |

Nach dem Anwachsen der Kultur, das heißt nach etwa 6 bis 8 Stunden, wird Glukose nachdosiert. Die zugegebene Glukosemenge richtet sich nach dem Glukoseverbrauch und der Säurebildung. Insgesamt wird innerhalb von 40 bis 50 Stunden eine Glukosemenge zugegeben, die etwa 10 Gew.-% des Nährmediums ausmacht. Aus der Glukose werden 110 bis 115 g D-Milchsäure/l des Kulturmediums gebildet (gleich 159 bis 166 g Calciumlactat). Die Isolierung der freien Milchsäure erfolgt nach bekannten Verfahren, nämlich der Calciumfällung mit Schwefelsäure, Filtration, Eindampfen und Reinigung durch Destillation als Ethyl- oder Methylester.

Die Verwendung des Mediums 3 hat den Vorteil, daß im Endprodukt keine nachweisebare Menge L-Milchsäure enthalten ist. Das Medium 4 bietet dafür den Vorteil, daß es nach der Fermentation deutlich besser filtrierbar ist als das Medium 3, da das Sojamehl im Medium 3 nur teilweise abgebaut wird. Ein mit Medium 4 hergestelltes Produkt enthält 1,5 bis 2% L-Milchsäure, welches aus dem Cornsteep im Medium stammt.

Nach der Filtration ist eine Spaltung des Anteils der L-Milchsäure möglich, in dem das Filtrat über einen Enzymreaktor geleitet wird, welcher ein Enzym enthält, das spezifisch L-Milchsäure spaltet. Geeignet sind Systeme mit L-Lactatoxidase, L-Lactatdehydrogenase oder Cytochrom $b_2$. Die L-Lactatdehydrogenase benötigt als Cofaktor NAD, welches ebenfalls trägergebunden sein kann und dann regenerierbar und wieder verwendbar ist. Für Cytochrom $b_2$ ist Hexacyanoferrat als Cofaktor geeignet, welches mit Hilfe eines elektrischen Stroms an einer Edelmetallelektrode wieder reoxidiert werden kann.

Beispiel 3

Gemäß der in Beispiel 2 angegeben Arbeitsweise werden Stammkultur und Inokulum hergestellt, jedoch als Nährmedium das folgende Medium 5 verwendet, welches als zu vergärendes Substrat Milchzucker enthält (Angaben in g/l):

| | |
|---|---|
| Molkepulver | 60 |
| $CaCO_3$ | 70 |
| Hefeextrakt | 3 |
| Sojamehl | 5 |
| Natriumacetat | 5 |
| nichtionisches Tensid | 1 ml/l |

Wie im Beispiel 2 wird nach dem Anwachsen der Kultur das Zuckersubstrat nachdosiert und zwar so lange, bis die insgesamt zugefügte Milchzuckermenge 130 g/l Medium betrug. Die Milchsäurebildung dauerte etwas länger als im Beispiel 2, war aber nach 45 bis 55 Stunden beendet. Im übrigen wurde in gleicher Weise wie in Beispiel 2 verfahren.

Beispiel 4

Die Stammkultur und das Inokulum wurden unter Stickstoffüberlagerung wie unter Beispiel 1 angesetzt. Die Medien 3, 4 oder 5 wurden verwendet, jedoch enthielten sie zunächst kein $CaCO_3$. Dieses

4

wurde in trockener Form oder als eine 20 %ige Aufschlämmung nachdosiert, so daß der pH-Wert niemals unter 5,5 absank. Nach 48 Stunden wurde mit der Nachdosierung der Nährlösung begonne. Die Verdünnungsrate betrug zunächst 0,01 l/h pro Liter Nährlösung, wobei die gleiche Flüssigkeitsmenge kontinuierlich dem Fermenter entnommen wurde. Die Verdünnungsrate wurde langsam gesteigert, bis auf einen Wert von 0,03 bis 0,04 l/h pro Liter Nährlösung, wobei der Ausfluß aus dem Fermenter einen D-Milchsäuregehalt von 5 bis 7% aufwies. Die Zellmasse wurde aus dem Ausfluß mit Hilfe eines kontinuierlichen Separators abgetrennt und zu 90% in den Fermenter zurückgegeben. Der Überstand wurde mit Calciumhydroxid auf pH 6,5 eingestellt und auf etwa 1/10 seines ursprünglichen Volumens eingedampft. In einer Kühlfalle wurde das Konzentrat auf +4°C abgekühlt, wobei das Calciumlactat auskristallisierte. Die konzentrierte Lösung floß in die Kühlfalle kontinuierlich ein, während das ausgefallene Calciumlactat in regelmäßigen Abständen mit Hilfe eines Schiebers herausbefördert wurde. Die Mutterlauge wurde in den Fermenter zurückgeführt. Die Freisetzung der Milchsäure aus dem gewonnen Calciumlactat erfolgte mit Schwefelsäure und nachfolgender Filtration.

Beispiel 5

Die kontinuierliche Milchsäureherstellung gemäß Beispiel 4 wurde wiederholt mit dem Unterschied, daß zur pH-Steuerung nicht CaCO₃, sondern Natriumhydroxid verwendet wurde. Der Vorteil dieses Verfahrens liegt darin, daß es dann möglich ist, den pH auf 6,5 bis 6,8 zu halten, wodurch die Gärungsgeschwindigkeit von Lactobacillus bulgaricus DSM 2129 erhöht wird. Die entstandene Lösung von Natriumlactat wird über Ionenaustauschersäulen gegeben, die die Milchsäure adsorbieren. Sobalt eine Säule mit Milchsäure beladen ist, wird sie mit Salzsäure eluiert. Nach Regeneration mit verdünnter Natronlauge kann die Säule erneut zur Milchsäureadsorption eingesetzt werden.

**Patentansprüche für die Vertragsstaaten: BE, CH, DE, FR, GB, IT, LI, LU, NL, SE**

1. Verfahren zur Herstellung von D-Milchsäure durch anaerobe Fermentation eines Glukose und/oder Milchzucker und andere übliche Zusatzstoffe enthaltenden Nährmediums, dadurch gekennzeichnet, daß man als Milchsäure erzeugenden Mikroorganismus Lactobacillus bulgaricus DSM 2129 einsetzt.

2. Verfahren nach Anspruch 1, dadurch gekennzeichnet, daß man als Nährmedium Molke oder eine Suspension von Molkepulver einsetzt.

3. Verfahren nach Anspruch 2, dadurch gekennzeichnet, daß dem Nährmedium Glukose zugesetzt wird.

4. Verfahren nach Anspruch 1 bis 3, dadurch gekennzeichnet, daß man die Fermentation bei Temperaturen von 30 bis 50°C durchführt.

5. Verfahren nach Anspruch 1 bis 4, dadurch gekennzeichnet, daß man die Fermentation bei 40 bis 45°C durchführt.

6. Verfahren nach Anspruch 1 bis 5, dadurch gekennzeichnet, daß man die Fermentation bei einem pH-Wert von 4,5 bis 7 durchführt.

7. Verfahren nach Anspruch 1 bis 6, dadurch gekennzeichnet, daß man die Fermentation bei einem pH-Wert von 6,5 bis 6,8 durchführt.

8. Verfahren nach Anspruch 1 bis 7, dadurch gekennzeichnet, daß man dem Nährmedium Calciumcarbonat zusetzt.

9. Verfahren nach Anspruch 1 bis 7, dadurch gekennzeichnet, daß man unter einer Schutzgasatmosphäre arbeitet.

10. Lactobacillus bulgaricus DSM 2129.

**Patentansprüche für den Vertragsstaat Österreich:**

1. Verfahren zur Herstellung von D-Milchsäure durch anaerobe Fermentation eines Glukose und/oder Milchzucker und andere übliche Zusatzstoffe enthaltenden Nährmediums, dadurch gekennzeichnet, daß man als Milchsäure erzeugenden Mikroorganismus Lactobacillus bulgaricus DSM 2129 einsetzt.

2. Verfahren nach Anspruch 1, dadurch gekennzeichnet, daß man als Nährmedium Molke oder eine Suspension von Molkepulver einsetzt.

3. Verfahren nach Anspruch 2, dadurch gekennzeichnet, daß dem Nährmedium Glukose zugesetzt wird.

4. Verfahren nach Anspruch 1 bis 3, dadurch gekennzeichnet, daß man die Fermentation bei Temperaturen von 30 bis 50°C durchführt.

5. Verfahren nach Anspruch 1 bis 4, dadurch gekennzeichnet, daß man die Fermentation bei 40 bis 45°C durchführt.

6. Verfahren nach Anspruch 1 bis 5, dadurch gekennzeichnet, daß man die Fermentation bei einem pH-Wert von 4,5 bis 7 durchführt.

7. Verfahren nach Anspruch 1 bis 6, dadurch gekennzeichnet, daß man die Fermentation bei einem pH-Wert von 6,5 bis 6,8 durchführt.

8. Verfahren nach Anspruch 1 bis 7, dadurch gekennzeichnet, daß man dem Nährmedium Calciumcarbonat zusetzt.

**0 072 010**

9. Verfahren nach Anspruch 1 bis 7, dadurch gekennzeichnet, daß man unter einer Schutzgas-atmosphäre arbeitet.

**Revendications pour les Etats Contractants: BE, CH, DE, FR, GB, IT, LI, LU, NL, SE**

1. Procédé pour la préparation d'acide D-lactique par fermentation anaérobie d'un milieu nutritif contenant du glucose et/ou du lactose et d'autres additifs usuels, caractérisé en ce que, comme microorganisme produisant de l'acide lactique, on ajoute Lactobacillus bulgaricus DSM 2129.

2. Procédé selon la revendication 1, caractérisé en ce que comme milieu nutritif, on utilise le petitlait ou une suspension de poudre de lait.

3. Procédé selon la revendication 2, caractérisé en ce qu'on ajoute du glucose au milieu nutritif.

4. Procédé selon l'une quelconque des revendications 1 à 3, caractérisé en ce qu'on effectue la fermentation à des températures comprises entre 30 et 50°C.

5. Procédé selon l'une quelconque des revendications 1 à 4, caractérisé en ce qu'on effectue la fermentation entre 40 et 45°C.

6. Procédé selon l'une quelconque des revendications 1 à 5, caractérisé en ce qu'on effectue la fermentation à une valeur de pH comprise entre 4,5 et 7.

7. Procédé selon l'une quelconque des revendications 1 à 6, caractérisé en ce qu'on effectue la fermentation à une valeur de pH comprise entre 6,5 et 6,8.

8. Procédé selon l'une quelconque des revendications 1 à 7, caractérisé en ce qu'on ajoute du carbonate de calcium au milieu nutritif.

9. Procédé selon l'une quelconque des revendications 1 à 7, caractérisé en ce qu'on travaille sous atmosphère de gaz protecteur.

10. Lactobacillus bulgaricus DSM 2129.

**Revendications pour l'Etat Contractant: AT**

1. Procédé pour la préparation d'acide D-lactique par fermentation anaérobie d'un milieu nutritif contenant du glucose et/ou du lactose et d'autres additifs usuels, caractérisé en ce que comme microorganisme produisant de l'acide lactique, on utilise Lactobacillus bulgaricus DSM 2129.

2. Procédé selon la revendication 1, caractérisé en ce que comme milieu nutritif, on utilise le petitlait ou une suspension de laiten poudre.

3. Procédé selon la revendication 2, caractérisé en ce qu'on ajoute du glucose au milieu nutritif.

4. Procédé selon l'une quelconque des revendications 1 à 3, caractérisé en ce qu'on effectue la fermentation à des températures de 30 à 50°C.

5. Procédé selon l'une quelconque des revendications 1 à 4, caractérisé en ce qu'on effectue la fermentation entre 40 et 45°C.

6. Procédé selon l'une quelconque des revendications 1 à 5, caractérisé en ce qu'on effectue la fermentation à une valeur de pH comprise entre 4,5 et 7.

7. Procédé selon l'une quelconque des revendications 1 à 6, caractérisé en ce qu'on effectue la fermentation à une valeur de pH comprise entre 6,5 et 6,8.

8. Procédé selon l'une quelconque des revendications 1 à 7, caractérisé en ce qu'on ajoute du carbonate de calcium au milieu nutritif.

9. Procédé selon l'une quelconque des revendications 1 à 7, caractérisé en ce qu'on travaille sous atmosphère de gaz protecteur.

**Claims for the Contracting States: BE, CH, DE, FR, GB, IT, LI, LU, NL, SE**

1. A process for the production of D-lactic acid by anaerobic fermentation of a nutritive medium containing glucose and/or lactose and other usual additives, wherein Lactobacillus bulgaricus DSM 2129 is used as lactic acid producing microorganism.

2. The process as claimed in Claim 1, wherein whey or a suspension of whey powder is used as nutritive medium.

3. The process as claimed in Claim 2, wherein glucose is added to the nutritive medium.

4. The process as claimed in Claim 1, wherein the fermentation is carried out at temperatures of from 30 to 50°C.

5. The process as claimed in Claim 1, wherein the fermentation is carried out at 40 to 45°C.

6. The process as claimed in Claim 1, wherein the fermentation is carried out at a pH of from 4.5 to 7.

7. The process as claimed in Claim 1, wherein the fermentation is carried out at a pH of from 6.5 to 6.8.

8. The process as claimed in Claim 1, wherein calcium carbonate is added to the nutritive medium.

9. The process as claimed in Claim 1, wherein the operations are carried out under a protective gas atmosphere.

10. Lactobacillus bulgaricus DSM 2129.

6

**0 072 010**

**Claims for the Contracting State: AT**

1. A process for the production of D-lactic acid by anaerobic fermentation of a nutritive medium containing glucose and/or lactose and other usual additives, wherein Lactobacillus bulgaricus DSM 2129 is used as lactic acid producing microorganism.

2. The process as claimed in Claim 1, wherein whey or a suspension of whey powder is used as nutritive medium.

3. The process as claimed in Claim 2, wherein glucose is added to the nutritive medium.

4. The process as claimed in Claim 1, wherein the fermentation is carried out at temperatures of from 30 to 50°C.

5. The process as claimed in Claim 1, wherein the fermentation is carried out at 40 to 45°C.

6. The process as claimed in Claim 1, wherein the fermentation is carried out at a pH of from 4.5 to 7.

7. The process as claimed in Claim 1, wherein the fermentation is carried out at a pH of from 6.5 to 6.8.

8. The process as claimed in Claim 1, wherein calcium carbonate is added to the nutritive medium.

9. The process as claimed in Claim 1, wherein the operations are carried out under a protective gas atmosphere.